# EUROPÄISCHE PATENTSCHRIFT

(11) **EP 1 390 927 B1**
(45) Veröffentlichungstag und Bekanntmachung des Hinweises auf die Patenterteilung: **30.11.2005**
(21) Anmeldenummer: 02732420.1
(22) Anmeldetag: 07.05.2002
(51) Int. Cl.: G08B 29/14

(54) **MOBILER RAUCHGASERZEUGER UND VERFAHREN ZUM PRÜFEN EINES RAUCHGASMELDERS**
MOBILE FLUE GAS GENERATOR AND METHOD FOR TESTING A FLUE GAS INDICATOR
GENERATEUR DE GAZ DE FUMEE MOBILE ET PROCEDE DE TESTAGE D'UN INDICATEUR DE GAZ DE FUMEE

(30) Priorität: 09.05.2001 DE 10122572; 10.05.2001 US 290133 P; 18.05.2001 US 291880 P; 15.08.2001 DE 10139033; 01.10.2001 DE 103747 q; 09.01.2002 DE 10200584
(43) Veröffentlichungstag der Anmeldung: 25.02.2004
(73) Patentinhaber: Sata Limited, Welham Green, Hertfordshire, AL9 7JE (GB)
(72) Erfinder: KOCH, Hubert, 41199 Mönchengladbach (DE)
(74) Vertreter: Crawford, Andrew Birkby
(86) Internationale Anmeldenummer: PCT/DE2002/001650
(87) Internationale Veröffentlichungsnummer: WO 2002/091326

(56) Entgegenhaltungen:
- DE-A- 19 926 773
- FR-A- 2 583 553
- US-A- 3 729 979
- US-A- 5 170 148
- US-A- 5 611 620
- US-A- 5 644 071

## Beschreibung

Die Erfindung betrifft einen mobilen Rauchgaserzeuger zum Simulieren eines realen Rauchgases und ein Verfahren zum Prüfen eines Rauchgasmelders, bei dem ein in die Nähe des Rauchgasmelders gebrachter Rauchgaserzeuger mittels eines Prüfmediums ein Rauchgas erzeugt und das Rauchgas eine Prüfung des Rauchgasmelders einleitet.

Um Rauchgasmelder hinsichtlich ihrer Funktionstüchtigkeit zu überprüfen, sind unter anderem mobile Rauchgaserzeuger bekannt. Diese Rauchgaserzeuger werden an einen Rauchgasmelder gehalten, bis dieser durch das Rauchgas des Rauchgaserzeugers einen Alarm auslöst.

Hierbei gibt es Vorrichtungen, die ein Prüfaerosol in einer Druckflasche führen. Nachteilig bei diesen Vorrichtungen ist, dass sie unter anderem wegen der Druckflasche unvorteilhaft zu handhaben sind. Auch ein Auslösen einer solchen Vorrichtung mittels eines meist komplizierten und aufwändigen Auslösemechanismus ist unvorteilhaft durchzuführen.

Eine weitere Gattung von Prüfgeräten ist von der Firma Hekatron GmbH bekannt. Dieses Prüfgerät benutzt zur Rauchgasentwicklung ein Räucherstäbchen, welches in ein Gehäuse des Gerätes eingesetzt wird, wobei das Gehäuse mit großer Sorgfalt wieder verschlossen werden muss, da darauf zu achten ist, dass ein Dichtungsring, welcher einen Boden und ein Oberteil des Gehäuses abdichtet, nicht beschädigt wird. Bei der eigentlichen Prüfung des Rauchgasmelders wird dann eine Schlauchspitze des Prüfgerätes an den Rauchgasmelder gehalten, wobei das Rauchgas durch Kompression eines Gummiballs aus der Schlauchspitze heraus an den Rauchgasmelder gedrückt werden muss.

Neben der vorhergehend beschriebenen umständlichen Handhabung muss bei einem Pumpzyklus ein Belüftungsloch des Gehäuses manuell mit einem Finger verschlossen werden, sodass aus diesem Belüftungsloch kein Rauchgas während des Zusammendrückens des Gummiballs entweichen kann. Um den zusammengedrückten Gummiball wieder mit Luft zu füllen, wird nun das Belüftungsloch des Gehäuses freigegeben, sodass über dieses Frischluft in den Gummiball des Prüfgerätes zurückströmen kann. Ein solches Verfahren ist höchst umständlich, insbesondere dann, wenn die zu prüfenden Rauchgasmelder an einer Decke hoch aufgehängt sind.

Bekannte Prüfgeräte für Rauchgasmelder haben wie beschrieben meist eine recht komplizierte Bedienung. Hinzu kommt noch, dass die Prüfprozedur mit den bekannten Prüfgeräten wegen häufiger Fehlversuche nicht selten mehrmals durchgeführt werden müssen. Nachteilig ist es hierbei auch, dass durch einen längeren Verbleib der Prüfaerosole bzw. des Rauches des Rächerstäbchens in bzw. an dem Rauchgasmelder lange Wartezeiten entstehen, bis der Rauchgasmelder von einem Alarmzustand in den Normalzustand zurück gesetzt wird.

Die Erfindung hat die Aufgabe mobile Rauchgaserzeuger weiter zu entwickeln und hierdurch ein Prüfen eines Rauchgasmelders zu vereinfachen.

Die der Erfindung zugrunde liegende Aufgabe wird gelöst von einem mobilen Rauchgaserzeuger nach Anspruch 1.

Unter dem Begriff "Rauchgas" versteht man im vorliegenden Fall alle gasförmigen Medien, die insbesondere Feststoffpartikel aufweisen. Aber auch Gase, die explizit keine Feststoffpartikel aufweisen, werden im Sinne der Erfindung mit dem Begriff "Rauchgas" erfasst.

Dementsprechend umfasst der Begriff "Rauchgasmelder" alle technischen Einrichtungen, die ein Rauchgas mit Feststoffpartikeln sowie ein Rauchgas ohne Feststoffpartikeln detektieren können. Es versteht sich, dass die Erzeugung eines Rauchgases mit Feststoffpartikeln auch durch eine Erzeugung eines Gases ohne Feststoffpartikel ersetzbar ist. Dies ist insbesondere dann von Vorteil, wenn die Funktion eines Gasmelders überprüft werden soll, der auf das Vorhandensein eines Gases anspricht.

Besonders vorteilhaft ist es, wenn der mobile Rauchgaserzeuger ein elektrisches Gebläse aufweist. Das elektrische Gebläse kann hierbei ein kleiner Ventilator sein, der aus der Umgebung ein Luftvolumen ansaugt und dieses dann durch den mobilen Rauchgaserzeuger leitet, wobei das Luftvolumen das Rauchgas durch wenigstens eine Öffnung des mobilen Rauchgaserzeugers in Richtung eines Rauchgasmelders führt. Es ist möglich, anstatt des Ventilators jede andere Einrichtung zu verwenden, die in der Lage ist ein Luftvolumen zu beschleunigen. Besonders vorteilhaft ist es, wenn mit dem Gebläse ein Luftvolumen impulsbehaftet beschleunigt wird. Hierdurch kann das Rauchgas impulsweise und dadurch hoch konzentriert an einen Rauchgasmelder gebracht werden.

Eine weitere Ausführungsvariante sieht vor, dass der mobile Rauchgaserzeuger eine elektrische Energiequelle aufweist. Mittels dieser elektrischen Energiequelle ist beispielsweise eine Stromversorgung der Heizeinrichtung und des Gebläses gewährleistet. Als elektrische Energiequelle kann hierbei ein öffentliches Stromnetz oder eine Solarstromeinheit dienen. Bevorzugt besitzt die elektrische Energiequelle jedoch eine Batterie oder einen wiederaufladbaren Akkumulator. Insbesondere mittels der Batterie oder des Akkumulators ist der mobile Rauchgaserzeuger als stromunabhängiges Bauteil ausgebildet und unabhängig von einem Stromfestnetz.

Besonders vorteilhaft ist es, wenn der mobile Rauchgaserzeuger elektrisch auslösbar ist Hierbei wird lediglich dann ein Rauchgas erzeugt, wenn die Heizeinrichtung bzw. der Wärmeerzeuger elektrisch ausgelöst wird, wobei dann durch die Heizeinrichtung bzw. durch den Wärmeerzeuger ein Strom der elektrischen Energiequelle fließt.

Darüber hinaus ist vorgeschlagen, dass der mobile Rauchgaserzeuger einen Wärmeleitkörper aufweist. Beispielsweise ist der Wärmeleitkörper aus einem elektrisch leitenden Metallblech hergestellt, sodass sich durch die gegenüber der Oberfläche des Drahtes relativ große Oberfläche des Metallbleches die heizbare Oberfläche erhöht. Besonders vorteilhaft ist es, wenn der Wärmeleitkörper in Gestalt eines elektrischen Widerstands realisiert ist. Beispielsweise ist dieser Widerstand ein handelsüblicher Widerstand, sodass hierdurch ein Produkt aus der Massenfertigung zur Leistungssteigerung der Heizeinrichtung herangezogen werden kann.

Vorteilhaft ist es, wenn der Wärmeleitkörper einen porösen Körper aufweist. Die Porosität des Körpers erlaubt es, eine Substanz nach Art eines Schwammes aufzusaugen, so dass die aufgesaugte Substanz mit dem Wärmeleitkörper vorzugsweise im Bereich der Poren in einem innigen Kontakt mit dem porösen Körper steht. Wird hierbei der Wärmeleitkörper bzw. der elektrische Widerstand erhitzt, verdampft am porösen Körper angelagerte oder die in dem porösen Körper eingelagerte Substanz, wodurch das Rauchgas erzeugt wird.

Hat der Wärmeleitkörper selbst keinen porösen Körper, ist es vorteilhaft, wenn an dem Wärmeleitkörper ein poröses Bauteil angeordnet ist. Auch in diesem porösen Bauteil kann eine Substanz eingelagert und bei Bedarf verraucht werden.

Um beispielsweise eine Rauchgaserzeugung an einer gezielten Stelle des porösen Körpers bzw. des porösen Bauteils zu erzielen und/oder um ein unkontrolliertes Austreten der Substanz aus dem porösen Körper bzw. aus dem porösen Bauteil zu verhindern, ist es vorteilhaft, wenn der poröse Körper bzw. das poröse Bauteil eine Ummantelung aufweist, welche vorzugsweise als hitzebeständige Folie ausgebildet ist. Mittels der hitzebeständigen Folie wird die durch den porösen Körper bzw. durch das poröse Bauteil erhitzte Substanz daran gehindert, sich an einer unerwünschten Stelle des porösen Körpers bzw. des porösen Bauteils zu verflüchtigen.

Dementsprechend ist es vorteilhaft, wenn die Ummantelung wenigstens eine Öffnung aufweist, durch welche die erhitzte Substanz verdampft bzw. verraucht.

Gemäß einer weiteren Ausführungsvariante kann vorgesehen sein, dass der mobile Rauchgaserzeuger eine Wärmekammer aufweist, die zumindest teilweise mit einem Prüfmedium gefüllt ist.

Da es hinsichtlich Installations- oder Wartungsarbeiten schwierig sein kann, unter Druck stehende Patronen oder flüssigkeitsgefüllte Behälter zu transportieren, ist vorgeschlagen, dass das Prüfmedium einen Festkörper aufweist, welcher bei einer Erhitzung zumindest zum Teil verdampft bzw. verraucht. Dieser Festkörper kann ein Kunststoffelement oder ein Wachs sein. Das Wachs wird vorzugsweise durch ein sich bei Stromdurchfluss erwärmendes Widerstandselement erhitzt, so dass hierbei durch die Erwärmung zumindest ein Teil des Wachses bzw. des Festkörpers verdampft bzw. verraucht.

Das Prüfmedium kann fest oder flüssig sein, besonders vorteilhaft ist es, wenn das Prüfmedium ein gelartiges Material umfasst, welches bei der Erhitzung zumindest teilweise verdampft bzw. verraucht. Beispielsweise befindet sich in dem gelartigen Prüfmedium die elektrische Heizeinrichtung bzw. der Wärmeleitkörper, insbesondere der handelsübliche Widerstand, sodass bei der Erhitzung auf Grund der unmittelbaren Nahe der Heizeinrichtung ein Teil des Prüfmediums so stark erhitzt wird, dass es in einen gasförmigen Zustand verdampft bzw. verraucht und der mobile Rauchgaserzeuger hierbei ein Rauchgas erzeugt.

Das gelartige Material kann ein wasserklares Gel ohne Geruch sein. Gut geeignet sind Gele aus Kohlenwasserstoffen im Bereich der Weißöle, die unter Zusatz eines Gelbildners hergestellt sind. Derartige Gele haben vorzugsweise einen Siedepunkt, der über 250 °C liegt. Im vorliegenden Fall liegt der Schmelzpunkt vorzugsweise bei ca. 70 °C bis 90 °C. Dies führt dazu, dass bei derartigen Stoffen bei Raumtemperatur praktisch kein Verdampfen von Bestandteilen stattfindet. Daher kann auch bei der Lagerung der Stoffe oder aus dem im Prüfgerät befindlichen Vorrat eine Belastung der Umgebungsluft sicher ausgeschlossen werden.

In der Praxis werden bevorzugt langkettige aliphatische Kohlenwasserstoffe verwendet. Insbesondere wenn je Prüfvorgang eine Menge von etwa einem Milligramm verdampft wird, ist diese verdampfte Menge ohne gesundheitliche Relevanz, da aliphatische langkettige Kohlenwasserstoffe nur in hohen Konzentrationen zur mechanischen Reizung der oberen Atemwege führen können. Die beschriebenen Stoffe haben darüber hinaus den Vorteil, dass sie sich in der Nähe der Stoffabgabe absetzen und die freigesetzten Mengen an Material weder zur Korrosion noch zu anderen negativen Einflüssen auf benachbarten elektronischen oder mechanischen Bauteilen führen.

Aus diesem Grund kann eine derartige Substanz insbesondere bei allen in dieser Anmeldung beschriebenen Rauchgaserzeugern vorteilhaft eingesetzt werden.

Es wurde gefunden, dass es vorteilhaft ist, wenn das Prüfmedium lediglich eine Masse von weniger als 5 g, vorzugsweise von weniger als 1 g, aufweist. Vorteilhafterweise benötigt der erfindungsgemäße mobile Rauchgaserzeuger für eine Prüfsequenz lediglich ca. 0,001 g des gelartigen Prüfmediums, sodass mit dem erfindungsgemäßen mobilen Rauchgaserzeuger bis zu 600 Prüfungen durchgeführt werden, ohne den mobilen Rauchgaserzeuger mit einem Prüfmedium neu zu befüllen. Dies entspricht bei einem monatlichen Prüfzyklus einer Einsatzdauer von ca. 40 Jahren. Vorteilhafterweise lässt sich der mobile Rauchgaserzeuger mit einem neuen Prüfmedium nachfüllen. Durch die geringe Menge an Prüfmedium verringert sich das Gewicht des mobilen Rauchgaserzeugers erheblich.

Nach einer weiteren Ausgestaltung der Erfindung ist vorgesehen, dass die Heizeinrichtung mit dem Prüfmedium in Wirkkontakt steht. Hierdurch ist der Aufbau eines mobilen Rauchgaserzeugers sehr einfach konstruiert, wobei zusätzliche Mittel zur Rauchgasentwicklung überflüssig sind.

Bevorzugt ist in der Wärmekammer die Heizeinrichtung angeordnet, so dass sie sich vorteilhafter Weise in der unmittelbaren Umgebung des Prüfmediums befindet.

Um das erzeugte Rauchgas besonders effektiv und dynamisch aus der Wärmekammer hinaus zu befördern, ist es vorteilhaft, wenn in der Wärmekammer ein Gebläse angeordnet ist. Vorzugsweise ist das Gebläse an einer Lufteinlassöffnung der Wärmekammer bzw. in der Nähe einer Lufteinlassöffnung der Wärmekammer angeordnet, so dass bei aktiviertem Gebläse ein Luftstrom erzeugt wird, welcher durch die mit Rauchgas gefüllte Wärmekammer geleitet wird. Hierbei gelangt das Rauchgas mit dem Luftstrom mittels einer Auslassöffnung aus der Wärmekammer in die unmittelbare Umgebung des Rauchgasmelders.

Es ist vorgeschlagen, dass die Heizeinrichtung beim Verrauchen eines Prüfmediums eine Temperatur von mehr als 80 °C, vorzugsweise eine Temperatur von mehr als 110 °C, aufweist.

Ebenso ist vorgeschlagen, dass die Heizeinrichtung beim Verrauchen eines Prüfmediums eine Temperatur von weiniger als 200 °C, vorzugsweise eine Temperatur von weniger als 160 °C, aufweist.

Besonders vorteilhaft verdampft bzw. verraucht das Prüfmedium bei ca. 150 °C.

Vorteilhaft ist es, wenn der mobile Rauchgaserzeuger eine Sammeleinrichtung aufweist, in welcher ein erzeugtes Rauchgas zumindest temporär angehäuft wird. Insbesondere kann die Wärmekammer des mobilen Rauchgaserzeugers eine derartige Sammeleinrichtung darstellen. Hierdurch ist der mobile Rauchgasmelder besonders einfach aufgebaut. Vorteilhafter Weise ist die Wärmekammer derart konzipiert, dass zuerst ein Rauchgas in einer geschlossenen bzw. nahezu geschlossenen Wärmekammer erzeugt und erst nach einem gewissen Zeitraum des Rauchgaserzeugens aus der Wärmekammer heraus gelangt.

Um einen Luftstrom durch die Sammeleinrichtung zu führen und so das erzeugte Rauchgas mit sich zu führen, ist es vorteilhaft, wenn die Sammeleinrichtung wenigstens eine Eintrittsöffnung und/oder wenigstens eine Austrittsöffnung aufweist. Vorzugsweise sind die Öffnungen hierbei derart dimensioniert bzw. gestaltet, dass ein erzeugtes Rauchgas ohne ein aktives Fördern nicht bzw. in einer zu vernachlässigbaren Menge aus der Sammeleinrichtung bzw. aus der Wärmekammer entweicht. Das aktive Fördern kann durch den erzeugten Luftstrom des Gebläses realisiert werden.

Eine Ausführungsvariante sieht vor, dass die Sammeleinrichtung wenigstens ein Mittel zum Verschließen aufweist. Dieses Mittel zum Verschließen ist beispielsweise eine einfache Klappe oder ein Ventil oder ähnliches. Vorzugsweise ist ein derartiges Mittel in einer oder in allen der vorstehend beschriebenen Öffnung bzw. Öffnungen angeordnet. Es ist hierbei nicht zwingend erforderlich, dass das Mittel zum Verschließen die Sammeleinrichtung zu 100 % abdichtet.

Vielmehr genügt es die Sammeleinrichtung derart zu verschließen, dass aus ihr ein erzeugtes Rauchgas beispielsweise ohne einen künstlich erzeugten Luftstrom oder ähnliches nicht oder nur in geringer Menge austreten kann.

Vorteilhaft ist es, wenn die Mittel zum Verschließen einen Draht aufweisen, dessen Gestalt temperaturabhängig ist. Mittels eines solchen Drahtes ist es möglich, beispielsweise eine Klappe derart zu betätigen, dass sie die Sammeleinrichtung verschließt bzw. im Gegensatz hierzu zumindest teilweise öffnet. Hierbei ist der Draht vorzugsweise der Gestalt, dass er durch einen elektrischen Strom und die damit einhergehende Temperaturerhöhung verkürzt bzw. verlängert wird.

Besonders vorteilhaft ist es, wenn die Mittel zum Verschließen einen Nitinol-Draht aufweisen. Nitinol-Draht zieht sich beispielsweise bei einem Durchströmen mit 2,5 Volt in Folge der daraus resultierenden Erwärmung zusammen und dehnt sich nach Abkühlen entsprechend wieder aus. Hierbei kann das Zusammenziehen des Nitinol-Drahtes vorteilhaft zum Öffnen der Klappe verwendet werden.

Es versteht sich, dass alternativ hierzu verständlicherweise jeder andere Aktor oder beispielsweise auch ein Linearmotor verwendet werden kann.

Es ist weiter vorgeschlagen, dass die Sammeleinrichtung eine Rauchgaszuflussöffnung aufweist. Mittels dieser Rauchgaszuflussöffnung ist es möglich, dass ein Rauchgas, welches nicht unmittelbar in der Sammeleinrichtung erzeugt wird, über diese Rauchgaszuflussöffnung in die Sammeleinrichtung gelangt. Beispielsweise wird das Rauchgas in der Wärmekammer des mobilen Rauchgaserzeugers erzeugt und gelangt über die Rauchgaszuflussöffnung in die Sammeleinrichtung.

Besonders vorteilhaft ist es, wenn die Sammeleinrichtung einen Rauchgassensor und/oder einen Gassensor aufweist. Um den Rauchgaserzeuger hinsichtlich einer Rauchgaserzeugung zu überprüfen oder um festzustellen, ob eine genügend große Menge an Rauchgas erzeugt wurde und in der Sammeleinrichtung vorliegt, ist es vorteilhaft, wenn der entsprechende Rauchgassensor unmittelbar in der Sammeleinrichtung des Rauchgaserzeugers angeordnet ist. Beispielsweise wird durch ein Aktivieren des Rauchgassensors einem Benutzer des Rauchgaserzeugers optisch bzw. akustisch angezeigt, zu welchem Zeitpunkt genügend Rauchgas zum Prüfen eines Rauchgasmelders zur Verfügung steht, so dass der Benutzer, nachdem er den Rauchgaserzeuger in die Nähe eines Rauchgasmelders gebracht hat, einen Auslöser betätigt, welcher den Auslass der Sammeleinrichtung bzw. der Wärmekammer öffnet.

Besonders vorteilhaft ist es, wenn die Sammeleinrichtung einen veränderlichen Querschnitt aufweist. Mittels des veränderlichen Querschnittes werden beispielsweise unterschiedliche Drücke und Strömungen innerhalb der Sammeleinrichtungen bewirkt, was sich positiv auf die Verbreitung des Rauchgases auswirkt.

Eine baulich einfache Variante sieht vor, dass die Sammeleinrichtung einen Diffusor aufweist. Mittels des Diffusors können in der Sammeleinrichtung unterschiedliche Strömungsgeschwindigkeiten sowie unterschiedliche Drücke hervorgerufen werden.

Eine bevorzugte Ausführungsform sieht vor, dass die Sammeleinrichtung eine Venturi-Düse aufweist. Mittels der Venturi-Düse können in der Sammeleinrichtung ebenfalls unterschiedliche Drücke und Strömungsgeschwindigkeiten gezielt erreicht und eingesetzt werden.

Eine bevorzugte Ausführungsvariante sieht vor, dass der mobile Rauchgaserzeuger ein Gehäuse aufweist, welches zumindest teilweise elastisch ist. Insbesondere im Bereich der Wärmekammer bzw. der Sammeleinrichtung ist es vorteilhaft, wenn das Gehäuse aus einem duktilen Material besteht, welches sich beispielsweise durch eine Pumpbewegung leicht zusammen drücken lässt und hierdurch das Volumen der Wärmekammer bzw. der Sammeleinrichtung reduziert. Durch eine derartige Volumenreduzierung wird ein erzeugtes Rauchgas regelrecht aus der Wärmekammer bzw. aus der Sammeleinrichtung herausgestoßen, so dass ein möglichst großes und dichtes Rauchgasvolumen innerhalb kürzester Zeit bis an den Rauchgasmelder gelangt.

Eine vorteilhafte Weiterbildung der Erfindung besteht darin, dass an dem elastischen Gehäuse eine Vorratseinrichtung und/oder eine Dosiereinrichtung sowie ein Gebläse angeordnet sind. Derartige Einrichtungen werden beispielsweise über eine formschlüssige oder eine reibschlüssige Verbindung an dem Gehäuse angeordnet.

Vorteilhafter Weise sind insbesondere diese Einrichtungen in das Gehäuse des mobilen Rauchgaserzeugers eingesteckt. Beispielsweise ist das Gehäuse derart konzipiert, dass die eingesteckten Einrichtungen zusätzlich noch mittels einer Gummilippe an dem Gehäuse fixiert sind. Diese Gummilippe umschließt die eingesteckte Einrichtung zumindest teilweise, so dass die Einrichtung nur unter einem "bei Seite drücken" der Gummilippe aus dem Gehäuse entnommen werden kann.

Darüber hinaus übernimmt das elastische Gehäuse auch eine Schutzfunktion gegenüber Beschädigungen der Einrichtungen. Beispielsweise sind die in dem elastischen Gehäuse angeordneten Einrichtungen gegen Stöße hierdurch sehr gut geschützt, da das elastische Gehäuse zumindest einen Teil der Stoßenergie absorbieren kann.

Es versteht sich, dass die vorstehend erwähnten Einrichtungen neben einem Einstecken auch mittels eines Gewindes an bzw. in dem Gehäuse angeordnet werden können.

Das Gehäuse ist hierbei vorzugsweise aus einem Kunststoff bzw. aus einem Elastomer hergestellt, so dass es gegenüber anderen Gegenständen nur einen relativ weichen Widerstand aufweist. Hierdurch kann auch die Gefahr von Beschädigungen an anderen Gegenständen verringert werden.

Eine besonders bevorzugte Ausführungsvariante sieht vor, dass der mobile Rauchgaserzeuger wenigstens ein Aufnahmemittel zum Anordnen wenigstens eines Bauteils an dem mobilen Rauchgaserzeuger aufweist. Hierdurch lassen sich insbesondere die vorhergehend beschriebenen Einrichtungen in bzw. an das Gehäuse des mobilen Rauchgaserzeugers besonders einfach anordnen.

Vorteilhaft ist es, wenn in einem Aufnahmemittel eine Vorratseinrichtung, welche ein Prüfmedium bevorratet, angeordnet ist. Mittels des Aufnahmemittels kann die Vorratseinrichtung besonders leicht an dem bzw. in dem mobilen Rauchgaserzeuger angebracht werden. Hierdurch wird ein Austausch von Vorratseinrichtungen mit gegebenenfalls unterschiedlichen Prüfmedien sehr einfach gestaltet.

Um ein in der Vorratseinrichtung befindliches Prüfmedium auf einfache Art und Weise in die Nähe der Heizeinrichtung zu führen, ist es vorteilhaft, wenn das Aufnahmemittel, in welchem die Vorratseinrichtung angeordnet ist, mit einer Ausgabeeinrichtung zum Ausgeben eines Prüfmediums kommuniziert.

Die Ausgabeeinrichtung ist hierbei vorzugsweise in unmittelbarer Nähe der Heizeinrichtung angeordnet, so dass das durch die Ausgabeeinrichtung ausgehende Prüfmedium schnell mit der Heizeinrichtung in Kontakt tritt und hierbei verdampft bzw. verraucht. Darüber hinaus ist die Ausgabeeinrichtung vorteilhafter Weise derart beabstandet von der Heizeinrichtung angeordnet, dass die Heizeinrichtung die Ausgabeeinrichtung nicht durch einen ungünstigen Temperatureinfluss beschädigt.

Damit baulich besonders einfach gewährleistet ist, dass das Prüfmedium von der Vorratseinrichtung zur Ausgabeeinrichtung gelangt, ist es vorteilhaft, wenn das Gehäuse wenigstens eine Bohrung aufweist, die als Prüfmediumzufuhr genutzt wird, wobei die Prüfmediumzufuhr ein Aufnahmemittel der Vorratseinrichtung und eine Ausgabeeinrichtung für ein Prüfmedium miteinander verbindet.

Vorteilhaft ist es, wenn die Vorratseinrichtung ein Einwegbauteil ist. Hierdurch ist eine einfache Handhabung hinsichtlich eines Nachfüllvorgangs des Prüfmittels gewährleistet, da eine entleerte Vorratseinrichtung durch eine weitere Vorratseinrichtung ersetzt werden kann. Neben dieser einfachen und bevorzugten Ausführungsvarianten ist es auch möglich, die Vorratseinrichtung nach Aufbrauch des Prüfmittels nachzufüllen.

Eine Vorratseinrichtung ist baulich einfach gestaltet, wenn die Vorratseinrichtung einen Zylinder und einen Kolben aufweist. Insbesondere ist auf diese Weise eine Einwegvorratseinrichtung auf einfache Art und Weise hergestellt, wenn der Kolben bzw. die Kolbenstange kürzer ist als der eigentliche Zylinder. Somit kann bei vollständig eingedrücktem Kolben insbesondere die Kolbenstange nicht mehr aus dem Zylinder bewegt werden, wodurch ein Nachfüllen des Zylinders nahezu unmöglich ist bzw. nur mit einem hohen Aufwand durchgeführt werden kann. Insbesondere hierdurch ist ein unsachgemäßes Nachfüllen der Vorratseinrichtung unterbunden. Dadurch wird die Gefahr verringert, dass eine nicht für den mobilen Rauchgaserzeuger vorgesehene Substanz die Heizeinrichtung verunreinigt bzw. mittels ihr ein eventuell gesundheitsschädliches Rauchgas erzeugt wird.

Darüber hinaus ist es vorteilhaft, wenn in einem Aufnahmemittel eine Dosiereinrichtung angeordnet ist. Mittels der Dosiereinrichtung lässt sich eine aus der Vorratseinrichtung auszugebende Prüfmediummenge exakt dosieren. Somit ist der Verbrauch des Prüfmediums besonders genau einstellbar.

Die Vorratseinrichtung kann besonders einfach mit der Dosiereinrichtung kommunizieren, wenn die Dosiereinrichtung einen Haltebereich zum Aufnehmen der Vorratseinrichtung aufweist. Die Vorratseinrichtung kann hierbei entweder kraftschlüssig oder formschlüssig am Haltebereich der Dosiereinrichtung befestigt werden.

Darüber hinaus ist es vorteilhaft, wenn die Dosiereinrichtung ein Einstellmittel aufweist, welches vorzugsweise rastbar ist. Mit Hilfe des Einstellmittels der Dosiereinrichtung ist es möglich, besonders einfach eine vorbestimmte Menge eines Prüfmediums aus dem Vorratsbehälter durch die Ausgabeeinrichtung an die Heizeinrichtung abzugeben, so dass eine möglichst genau vorbestimmte Menge an Prüfmedium an der Heizeinrichtung verdampft bzw. verraucht wird.

Hierbei wird die Dosiereinrichtung vorzugsweise manuell verstellt, so dass beispielsweise der Kolben im Zylinder um einen Weg x bewegt wird. Es versteht sich, dass neben der manuellen Betätigung der Dosiereinrichtung auch eine elektrische bzw. eine elektronische Betätigung vorgesehen sein kann.

Darüber hinaus kann der mobile Rauchgaserzeuger ein Koordinationsmittel aufweisen, welches das Betätigen des Gebläses und das Betätigen der Dosiereinrichtung koordiniert. Beispielsweise ist der Betrieb des Ventilators und der Dosiereinrichtung elektronisch geregelt bzw. gesteuert. Vorzugsweise wird der Betriebszustand der Heizeinrichtung ebenfalls mittels des Koordinationsmittels gesteuert.

Hierbei ist es möglich, nach einem Startvorgang des Prüfens die Heizeinrichtung zuerst vorzuheizen, wobei anschließend, ca. nach zwei Sekunden, der Lüfter des Gebläses anläuft und einen Luftstrom erzeugt, der das erzeugte Rauchgas aus der Wärmekammer befördert.

Um beispielsweise genügend Prüfmittel an der Heizeinrichtung zur Verfügung zu haben, ist es vorteilhaft, wenn die Dosiereinrichtung automatisch Prüfmittel aus der Vorratseinrichtung in Richtung der Heizeinrichtung befördert. Dieses kann in Zyklen geschehen, die beispielsweise von der Leistung der Heizeinrichtung abhängen.

Darüber hinaus ist es möglich, das Gebläse gepulst zu betreiben, so dass hierbei Intervalle erzeugt werden, in welchen das Gebläse einen größeren Luftstrom bzw. in welchen der Lüfter einen demgegenüber geringeren Luftstrom erzeugt.

Ebenso ist es vorteilhaft, wenn die Heizeinrichtung gepulst mit Energie versorgt wird. Beispielsweise kann hiermit die Temperatur der Heizeinrichtung auf eine Verdampfungstemperatur tV = 150 °C gebracht und gehalten werden. Ansonsten besteht die Gefahr, dass eine zu starke Erhitzung der Heizeinrichtung Beschädigungen am mobilen Raucherzeuger hervorruft.

Unter dem Begriff "gepulst" versteht man im Sinne der Erfindung, dass eine Energiebereitstellung für die betroffenen Einrichtungen, vorzugsweise automatisch, variiert wird.

Darüber hinaus ist vorgeschlagen, dass der mobile Rauchgaserzeuger eine Kapillareinrichtung aufweist. Beispielsweise ist ein Ende eines Kapillarrohres in einem Gehäuse, in welchem sich das Prüfmedium befindet, angeordnet. Das andere Ende des Kapillarrohres weist dagegen eine Heizeinrichtung auf oder steht mit der Heizeinrichtung unmittelbar in Wirkverbindung. Durch das Kapillarrohr bewegt sich mittels Adhäsionskräften immer ein Teil des Prüfmediums in den Bereich der Heizeinrichtung, die bei einer Aktivierung für ein Verrauchen des Prüfmediums sorgt.

Eine andere Ausführungsvariante sieht vor, dass der Rauchgaserzeuger einen Fluidbehälter und eine Zündeinrichtung aufweist. Der Fluidbehälter kann auch als Überdruckbehälter so ausgebildet sein, dass er es erlaubt, das Fluid zu versprühen. Das Fluid kann in Zusammenwirken mit der Heizeinrichtung durch Verdunsten oder Verbrennen der Flüssigkeit ein Gas erzeugen, wobei dieses Gas dann durch eine Zündeinrichtung gezündet wird, so dass hierbei ein Rauchgas und/oder eine Hitze erzeugt wird.

Vorzugsweise ist die Heizeinrichtung femsteuerbar, sodass sie lediglich bei Bedarf eingeschaltet wird. Dies ist beispielsweise der Fall, wenn der erfindungsgemäße mobile Rauchgaserzeuger in unmittelbarer Nähe eines Rauchgasmelders gehalten wird. Eine derartige Fernsteuerung kann mittels einer Leitung oder kabellos ausgeführt sein und erlaubt es, den mobilen Rauchgaserzeuger zu beliebigen Zeiten auszulösen.

Um ein Überhitzen des Prüfmediums zu vermeiden, ist es vorteilhaft, wenn der mobile Rauchgaserzeuger eine Zeitschaltuhr aufweist. Hierdurch besteht die Möglichkeit, die Heizdauer der Heizeinrichtung temporär zu begrenzen, sodass für ein erneutes Aktivieren der Heizeinrichtung beispielsweise ein Schalter entsprechend betätigt werden muss.

Eine besonders bevorzugte Ausführungsvariante sieht vor, dass der Rauchgaserzeuger eine Schnittstelle zu einem Netzwerk aufweist. Beispielsweise ist der Rauchgaserzeuger an ein lokales Netzwerk eines Gebäudes angeschlossen, so dass der Rauchgaserzeuger von einer zentralen Einrichtung aus angesteuert werden kann. Die Schnittstelle kann hierbei sowohl kabelgebunden als auch kabellos ausgebildet sein. Es ist ebenfalls möglich, dass der Rauchgaserzeuger nicht nur zu einem lokalen Netzwerk einen Kontakt aufweist, sondern vielmehr auch zu einem weiträumigen Netzwerk. Beispielsweise ist ein Rauchgaserzeuger über ein weiträumiges Netzwerk mit einem zentralen Sicherheitsdienst verbunden, welcher sich nicht unmittelbar in dem Gebäude des zu prüfenden Rauchgasmelders befindet.

Die Aufgabe der Erfindung wird ebenfalls von einem Verfahren nach Anspruch 16 gelöst. Hierbei ist es besonders vorteilhaft, dass für die Erzeugung des Rauchgases nur eine sehr geringe Menge eines Prüfmediums verbraucht wird.

Vorteilhaft ist es auch, dass das Rauchgas durch ein elektrisches Gebläse des mobilen Rauchgaserzeugers unmittelbar in bzw. an den Rauchgasmelder geblasen wird, sodass ein gezieltes Berauchen des Rauchgasmelders geschieht, wobei unter anderem durch die Zielstrebigkeit des Verfahren die Effektivität einer Rauchgasmelderprüfung wesentlich erhöht wird.

Ist die Prüfsequenz des Rauchgasmelders erfolgreich eingeleitet, ist es besonders vorteilhaft, wenn der Rauchgasmelder unmittelbar nach dem Berauchen nachgelüftet wird, sodass die Prüfung schnellstmöglich beendet wird. Durch dieses gezielte Nachbelüften mittels des erfindungsgemäßen mobilen Rauchgaserzeugers wird die Gefahr einer unerwünschten Ablagerung von Rauchgaspartikeln in dem Rauchgasmelder, aber auch innerhalb des mobilen Rauchgaserzeugers, verringert.

Weiterhin ist es vorteilhaft, dass durch das aktive Belüften des Rauchgasmelders das gesamte Prüfungsprozedere wesentlich beschleunigt wird, wodurch unter anderem mehr Rauchgasmelder pro Zeit geprüft werden können.

Eine weiterführende Ausgestaltung sieht vor, dass das erzeugte Rauchgas gesammelt wird, bevor es aus den mobilen Rauchgaserzeuger gelangt. Vorteilhafter Weise wird erst ein gewisses Volumen an Rauchgas gesammelt, welches ausreicht, einen Rauchgasmelder bzw. einen Gasmelder zu aktivieren. Eine andere Verfahrensvariante sieht vor, das zum Fördern des Rauchgases ein veränderlicher Volumenstrom erzeugt wird.

Vorteilhaft ist es, wenn der Rotor des Gebläses hierzu die Umdrehungszahl ändert.

Dies kann besonders einfach realisiert werden, wenn dem Gebläse insbesondere während des Prüfens diskontinuierlich Energie zur Verfügung gestellt wird.

Um die Heizeinrichtung auf eine gewünschte Temperatur einzustellen und auf dieser zu halten, ist es vorteilhaft, wenn die Heizeinrichtung insbesondere während des Prüfens diskontinuierlich Energie zur Verfügung gestellt bekommt.

Damit ein Prüfmedium optimal verdampft bzw. verraucht werden kann, ist es vorteilhaft, wenn die Heizeinrichtung vor einem Zuführen eines Prüfmediums vorgewärmt wird.

Eine weitere Verfahrensvariante sieht vor, dass mittels der erzeugten Hitze der Heizeinrichtung die Funktion eines Hitzemelders geprüft wird. Vorzugsweise wird dies durchgeführt, ohne ein Rauchgas zu erzeugen. Hierbei kann der mobile Rauchgaserzeuger ebenfalls vorteilhaft zum Simulieren einer Hitze verwendet werden, so dass mit dem erfindungsgemäßen Rauchgaserzeuger nicht nur ein Rauchgasmelder bzw. ein Gasmelder, sondern darüber hinaus auch ein Hitzemelder geprüft werden kann. Somit sind mittels einer einzigen mobilen Prüfeinrichtung nahezu alle Feuermelder zu prüfen, so dass hierdurch Investitionskosten hinsichtlich unterschiedlicher Prüfeinrichtungen eingespart werden können. Besonders vorteilhaft ist es, wenn hierbei die erzeugte Hitze unterstützend mittels des aktivierten Gebläses zu einem Hitzemelder hin geblasen wird.

Es versteht sich in diesem Zusammenhang, dass die Kombination des Hitzerzeugers und des Gebläses, insbesondere deren gemeinsame Verwendung zum Prüfen eines Hitzeerzeugers auch unabhängig von den übrigen Merkmalen der Erfindung erfindungswesentlich sind.

Vorteilhaft ist es, wenn mittels einer zentralen Überwachungseinrichtung der mobile Rauchgaserzeuger aktiviert wird, wobei das erzeugte Rauchgas von einem Rauchgasmelder detektiert wird, und der Rauchgasmelder hierdurch aktiviert wird, wobei der Rauchgasmelder ein Datensignal übermittelt.

Unter einer Überwachungseinrichtung versteht man beispielsweise eine zentrale Einrichtung, in welcher die sicherheitsrelevanten Funktionen eines Gebäudes überwacht werden.

Eine bevorzugte Verfahrensvariante sieht vor, dass das Datensignal an eine zentrale Überwachungseinrichtung und/oder an eine Notfalleinrichtung übermittelt wird. Beispielsweise ist die zentrale Überwachungseinrichtung unmittelbar in einem Gebäude vorhanden, so dass alle zur Prüfung notwendigen Schritte von dort aus koordiniert werden können.

Vorteilhaft ist es auch, wenn das Datensignal zusätzlich oder ausschließlich an eine Notfalleinrichtung übermittelt wird. Eine Notfalleinrichtung ist beispielsweise eine ortsansässige Feuerwehr oder ein sonstiger Rettungsdienst.

Falls das Datensignal parallel zu der zentralen Überwachungseinrichtung auch an die Notfalleinrichtung übermittelt wird, ist es von Vorteil, wenn der Überwachungseinrichtung bzw. der Notfalleinrichtung vor dem Prüfen eine Information über das bevorstehende Prüfen übermittelt wird. Beispielsweise enthält die Information Daten darüber, an welchem Tag und zu welcher Uhrzeit die Prüfung stattfindet sowie über die Art der Prüfeng und über die Dauer der Prüfung. Vorzugsweise wird die Notfalleinrichtung automatisch von der zentralen Überwachungseinrichtung informiert.

Weitere Vorteile, Ziele und Eigenschaften vorliegender Erfindung werden anhand nachfolgender Erläuterung anliegender Zeichnung beschrieben, in welcher beispielhaft ein mobiler Rauchgaserzeuger dargestellt ist.

Es zeigt
- Figur 1: eine zweigeteilte Wärmekammer,
- Figur 2: eine teilweise geschnittene Wärmekammer,
- Figur 3: schematisch eine Akku-Stromquelle,
- Figur 4: einen Widerstand und eine Manschette mit Kapillarflächen,
- Figur 5: eine schematische Darstellung eines mobilen Rauchgaserzeugers, die der vorliegenden Erfindung Hintergrund verleiht,
- Figur 6: einen mobilen Rauchgaserzeuger in Wechselwirkung mit einem Rauchgasmelder,
- Figur 7: einen Längsschnitt durch einen mobilen Rauchgaserzeuger gemäß einer bevorzugten Ausgestaltung der vorliegenden Erfindung,
- Figur 8: eine perspektivische Ansicht des Gehäuses des in Fig. 7 gezeigten mobilen Rauchgaserzeugers,
- Figur 9: schematisch eine Vorderansicht auf ein alternatives Gehäuse eines weiteren mobilen Rauchgaserzeugers,
- Figur 10: schematisch eine Seitenansicht auf das alternative Gehäuse aus der Figur
- Figur 11: schematisch eine Rückansicht auf das alternative Gehäuse aus den Figuren 9 und 10,
- Figur 12: schematisch eine Draufsicht auf das alternative Gehäuse aus den Figuren 9 bis 11 und
- Figure 13: schematisch eine perspektivische Ansicht auf das alternative Gehäuse aus den Figuren 9 bis 12.

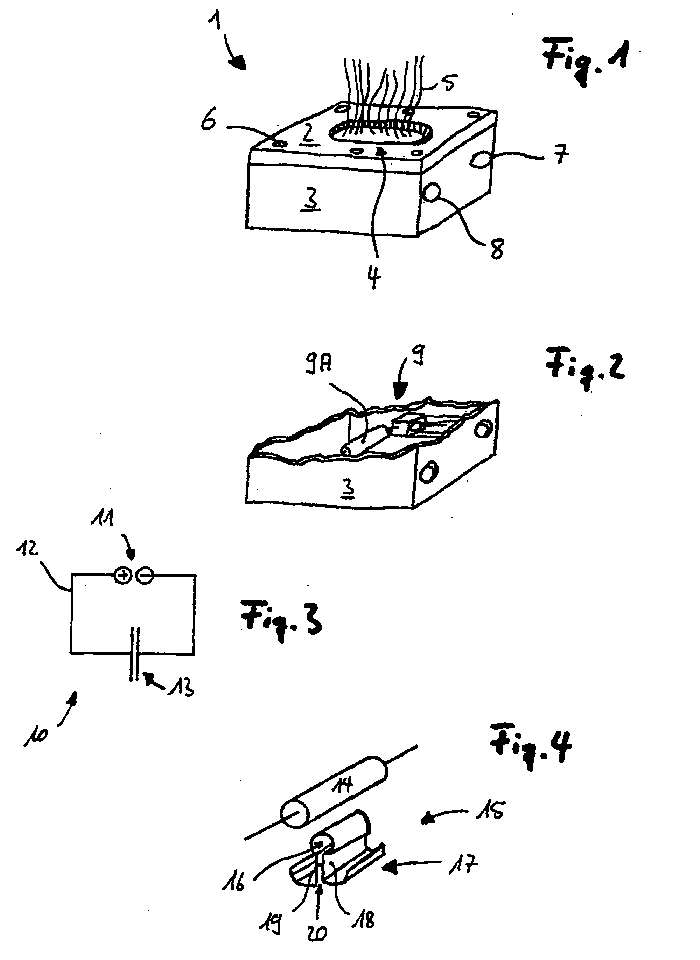

Es ist zu beachten, dass die Figuren 1 bis 5 und die dazugehörige Beschreibung als ein zum Verständnis der Erfindung nützlicher Hintergrund bereitgestellt werden und nicht Teil der beanspruchten Erfindung bilden.

Die in Figur 1 zweigeteilte Wärmekammer 1 weist einen Verschlussdeckel 2 und ein Basisgehäuse 3 auf. Der Verschlussdeckel 2 hat in seiner Mitte eine Öffnung 4, durch welche ein erzeugtes Rauchgas 5 aufsteigt. Der Verschlussdeckel 2 ist mittels einer Vielzahl von Schrauben 6 (hier nur exemplarisch beziffert) mit dem Basisgehäuse 3 verschraubt.

Das Basisgehäuse hat an einer Seite zwei Bohrungen 7 und 8, welche Steckkontakte für einen elektrischen Anschluss (hier nicht dargestellt) aufnehmen.

Die Figur 2 zeigt das Basisgehäuse 3 der zweigeteilten Wärmekammer 1 in einem Aufbruch. Im Inneren des Basisgehäuses 3 ist eine Heizeinrichtung 9 angeordnet. Die Heizeinrichtung 9 hat einen Widerstand 9A.

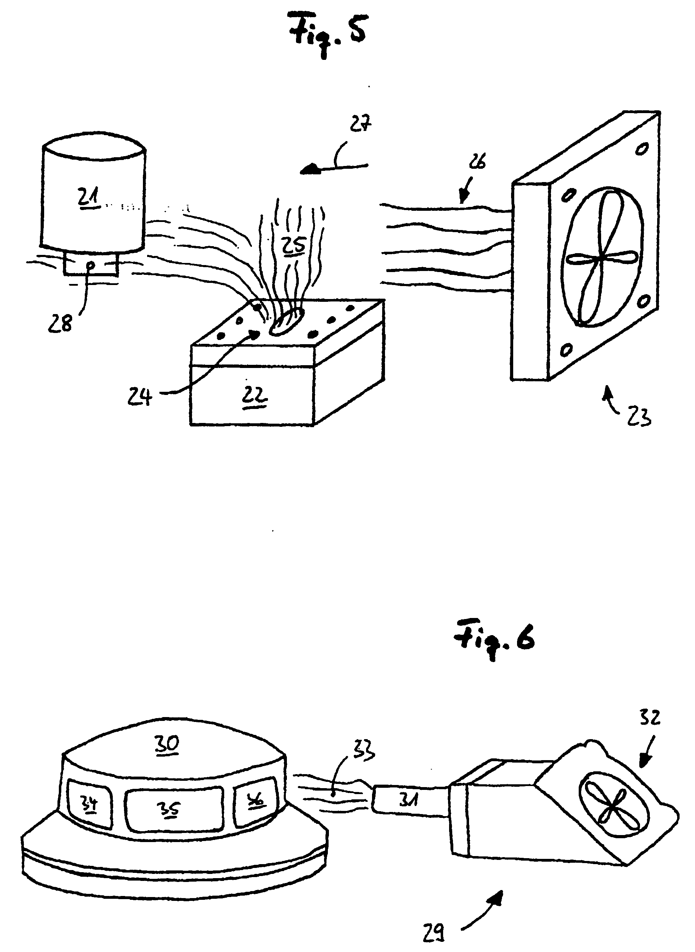

Die Figur 3 zeigt prinzipiell den einfachen Aufbau eines elektrischen Stromkreises 10 eines erfindungsgemässen mobilen Rauchgaserzeugers 29. Als Stromquelle dient hierbei ein Akkumulator 11, welcher mittels einer Drahtverbindung 12 mit einem Wärmeleitkörper 13 (Kondensator, ohmscher Widerstand) verbunden ist.

In Figur 4 sind ein ohmscher Widerstand 14 sowie eine Kapillarmanschette 15 gezeigt, wobei der Durchmesser des Widerstandes 14 dem Durchmesser der Kapillarmanschette 15 in einem Bereich 16 entspricht.

Mit seinem unteren Bereich 17 ist die Kapillarmanschette 15 in einem gelartigen Prüfmedium (hier nicht dargestellt) angeordenet, wobei sich das gelartige Prüfmedium im Innenbereich der Kapillarflächen 18 und 19 mittels Kapillarkräften in Pfeilrichtung 20 zwischen den beiden Kapillarinnenflächen 18 und 19 zu dem Widerstand 14 hinbewegt.

In der Darstellung nach Figur 5 ist die Anordnung eines Rauchgasmelders 21, einer Wärmekammer 22 und eines Ventilators 23 dargestellt. Durch eine Öffnung 24 der Wärmekammer 22 gelangt ein Rauchgas 25 in die Umgebung. Der Ventilator 23 bläst einen Luftstrom 26 in eine Pfeilrichtung 27. Hierbei wird das Rauchgas 25 mitgeführt und von einem Detektor 28 des Rauchgasmelders 21 registriert, wodurch ein Alarmsignal iniiziert wird. Ist die Prüfung des Rauchgasmelders 21 abgeschlossen, wird die Entwicklung des Rauchgases 25 in der Wärmekammer 22 unterbunden, in dem die elektrische Heizeinrichtung ausgeschaltet wird. Der Luftstrom 26 des Ventilators 23 bläst den Rachgasmelder 21, insbesondere den Detektor 28 des Rauchgasmelders 21, von restlichen Rauchgaspartikeln frei.

In Figur 6 ist ein mobiler Rauchgaserzeuger 29 in unmittelbarer Nähe zu einem Rauchgasmelder 30 gehalten. Hierbei weist der mobile Rauchgaserzeuger 29 in seinem vorderen Bereich ein Rohr 31 auf und in seinem hinteren Bereich einen Ventilator 32. Im Inneren des mobilen Rauchgaserzeugers 29 befindet sich eine vorhergehend beschriebene Wärmekammer 22 zur Rauchgaserzeugung. Ein durch den Ventilator 32 geförderter Luftstrom bläst ein in dem mobilen Rauchgaserzeuger erzeugtes Rauchgas 33 in Richtung des Rauchgasmelders 30. Der Rauchgasmelder 30 verfügt hierbei über Öffnungen 34, 35 und 36, durch die das Rauchgas 33 bis an einen Detektor des Rauchgasmelders gelangt. Mit dem Rohr 31 wird ein genaues Zielen zum Anströmen eines Rauchgasmelders 30 durch einen mobilen Rauchgaserzeugers 29 wesentlich erleichtert.

Der mobile Rauchgaserzeuger 37 (Figur 7) hat ein elastisches Gehäuse 38, in welchem eine Vorratseinrichtung 39 für ein Prüfmedium 50, eine Dosiereinrichtung 40, ein Gebläse 41, eine Sammeleinrichtung 42, eine Ausgabeeinrichtung 43 für das Prüfmedium 50 sowie eine Heizeinrichtung 44 angeordnet sind.

Insbesondere die Vorratseinrichtung 39, die Dosiereinrichtung 40 und das Gebläse 41 sind jeweils in einem Aufnahmemittel 45, 46 und 47 in dem Gehäuse 37 angeordnet, wobei das Aufnahmemittel 47 zusätzlich eine Gummilippe 48 aufweist. Die Aufnahmemittel 45, 46 und 47 sind derart gestaltet, dass in sie die Vorratseinrichtung 39, die Dosiereinrichtung 40 bzw. das Gebläse 41 schnell und einfach eingesteckt werden kann, so dass diese Bauteile lösbar in bzw. an dem elastischen Gehäuse 38 fixiert sind.

Das Aufnahmemittel 45 ist über eine Leitung 49 mit der Ausgabeeinrichtung 43 verbunden, so dass ein Prüfmedium 50 aus der Vorratseinrichtung 39 durch das elastische Gehäuse 37 zu der Ausgabeeinrichtung 43 gelangt, wobei die Ausgabeeinrichtung 43 das Prüfmedium 50 an die Heizeinrichtung 44 abgibt. Die Ausgabeeinrichtung 43 ist hierzu in unmittelbarer Nähe der Heizeinrichtung 44 angeordnet, so dass zum einen das aus der Ausgabeeinrichtung 43 austretende Prüfmedium 50 direkt mit der Heizeinrichtung 44 in Kontakt kommt und zum anderen die Heizeinrichtung 44 soweit von der Ausgabeeinrichtung 43 entfernt ist, dass die Ausgabeeinrichtung 43 durch eine Hitzeentwicklung der Heizeinrichtung 44 nicht beschädigt wird.

In diesem Ausführungsbeispiel besteht die Vorratseinrichtung 39 aus einem Zylinder 51, in welchem ein Kolben 52 eingebracht ist.

Mittels eines Einstellrädchens 40A der Dosiereinrichtung 40 wird eine Mechanik 40B der Dosiereinrichtung 40 derart eingestellt, dass die Mechanik 40B mit dem Kolben 52 der Vorratseinrichtung 39 kommuniziert und je nach Bedarf das Prüfmedium 50 in Pfeilrichtung 53 aus dem Zylinder 51 der Vorratseinrichtung 39 in die Leitung 49 drückt.

Um den Zylinder 51 der Vorratseinrichtung 39 sicher mit der Dosiereinrichtung 40 zu verbinden, hat die Dosiereinrichtung 40 einen Haltebereich 40C, in welchem die Vorratseinrichtung 39 eingesteckt ist.

Um einen Rauchgasmelder 30 (siehe Figur 6) optimal zu prüfen, wird die Heizeinrichtung 44 auf ca. 150 °C vorgeheizt, bevor das Prüfmedium 50 mit der Heizeinrichtung 44 in Kontakt kommt. Um insbesondere die Temperatur der Heizeinrichtung 44 zu regulieren, wird ein Energiefluss 54 durch die Heizeinrichtung 44 diskontinuierlich bereitgestellt.

Das an der Heizeinrichtung 44 verrauchte Prüfmedium 50 sammelt sich als Rauchgas 25 (siehe Figur 5) in der Sammeleinrichtung 42, bevor es mittels eines Luftvolumens 55, welches durch das aktivierte Gebläse 41 in die Sammeleinrichtung 42 eingesaugt wird, aus der Sammeleinrichtung 42 hinaus transportiert wird.

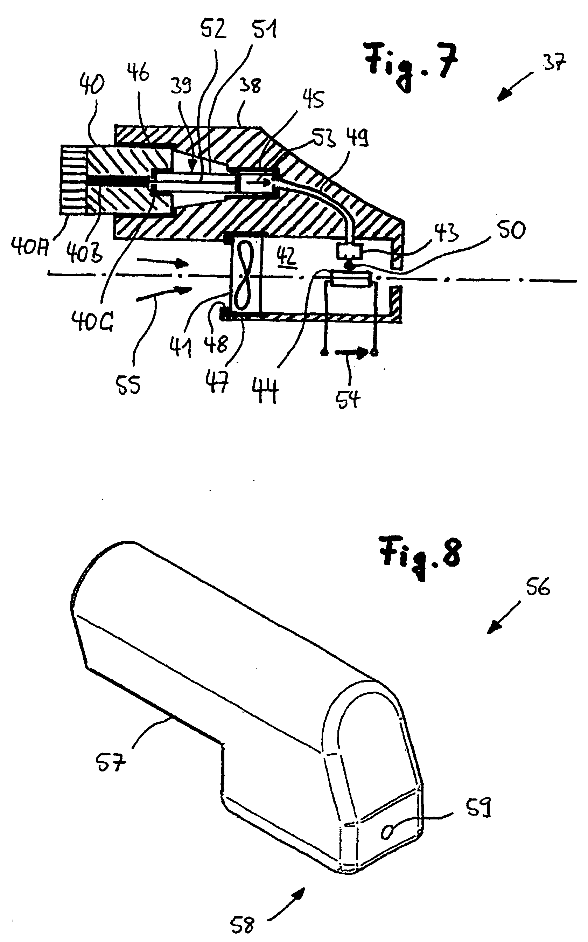

Das Ausführungsbeispiel 56 der Figur 8 zeigt ein elastisches Gehäuse 57 eines mobilen Rauchgaserzeugers, wobei das elastische Gehäuse 57 in seinem vorderen Bereich 58 eine Austrittsöffnung 59 hat, aus welcher ein erzeugtes Rauchgas 25 (siehe Figur 5) aus dem elastischen Gehäuse 57 heraus tritt.

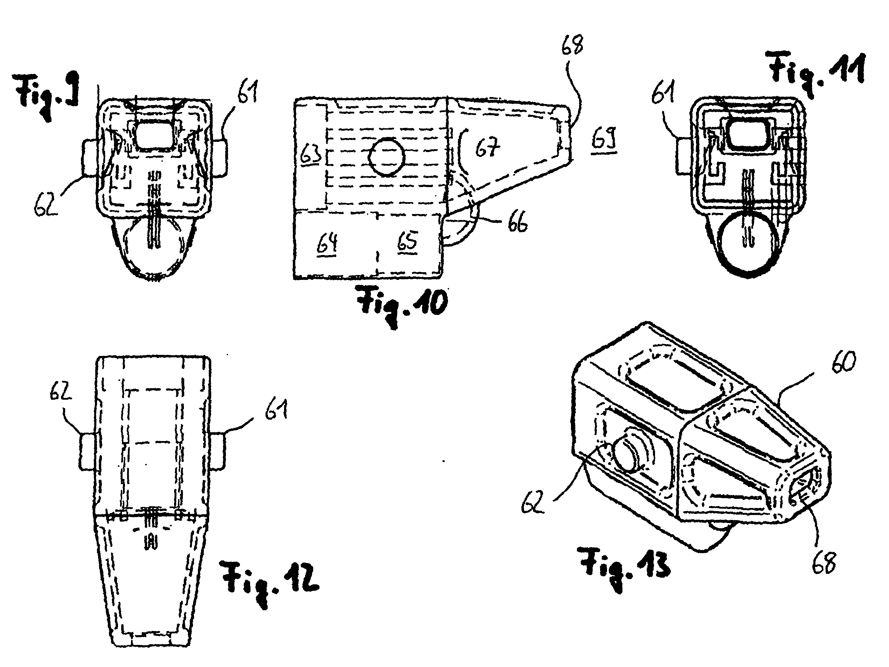

Das in den Figuren 9 bis 13 dargestellte elastische Gehäuse 60 umfasst zwei Haltebereiche 61 und 62, worüber das elastische Gehäuse 60 mit einer teleskopartigen Halteeinrichtung (hier nicht dargestellt) verbunden ist und beispielsweise an einen Rauchgasmelder 30 (siehe Figur 6) geführt wird.

Das elastische Gehäuse 60 umfasst darüber hinaus einen Bereich 63 in welchem ein Gebläse 41 (siehe Figur 7) angeordnet ist.

Eine Dosiereinrichtung 40 (siehe Figur 7) sowie eine Vorratseinrichtung 39 (siehe Figur 7) zum Bereitstellen eines Prüfmediums 50 (siehe Figur 7) sind in einem Bereich 64 und 65 des elastischen Gehäuses 60 angeordnet.

Das Prüfmedium 50 (siehe Figur 7) gelangt mittels einer Leitung 66 in einen Bereich 67 des elastischen Gehäuses 60, in welchem das Prüfmedium 50 (siehe Figur 7) verraucht wird. Das hierbei entstehende Rauchgas 25 (siehe Figur 5) gelangt mittels einer Ausgangsöffnung 68 in einen Ausgabebereich 69, in welchem ein Rauchgasmelder 30 (siehe Figur 6) angeordnet ist.

## Patentansprüche

1. Mobiler Rauchgaserzeuger, umfassend:
ein Gehäuse (38) mit einer elektrischen Heizeinrichtung (9, 44), einer Vorratsvorrichtung (39) und einer Gebläseeinrichtung (23, 32, 41), wobei die Vorratseinrichtung zum Zuführen eines Prüfmediums (50) zur Heizeinrichtung angeordnet ist, wobei die Heizeinrichtung zum Erhitzen des Prüfmediums zum Erzeugen eines Rauchgases (25, 33) angeordnet ist und die Gebläseeinrichtung die Aufgabe hat, für ein Umwälzen von Luft im Rauchgaserzeuger zu sorgen und das erzeugte Rauchgas durch eine Öffnung (24, 59, 68) aus dem Rauchgaserzeuger abzulassen.

2. Rauchgaserzeuger nach Anspruch 1, ferner umfassend eine Wärmekammer (42) zum Aufnehmen der Heizeinrichtung und der Gebläseeinrichtung, wobei die Wärmekammer eine Öffnung (59) aufweist.

3. Rauchgaserzeuger nach Anspruch 2, bei dem das erzeugte Rauchgas zumindest temporär in der Wärmekammer gesammelt wird.

4. Rauchgaserzeuger nach Anspruch 3, bei dem die Wärmekammer Mittel zum Öffnen und Schließen der Öffnung aufweist.

5. Rauchgaserzeuger nach Anspruch 1, ferner umfassend eine Sammelkammer zum zumindest temporären Sammeln des erzeugten Rauchgases.

6. Rauchgaserzeuger nach Anspruch 2, ferner umfassend eine Sammelkammer zum zumindest temporären Sammeln des erzeugten Rauchgases, wobei die Sammelkammer mit der Öffnung der Wärmekammer kommuniziert.

7. Rauchgaserzeuger nach Anspruch 5 oder 6, bei dem die Sammelkammer wenigstens eine Eintrittsöffnung und/oder wenigstens eine Austrittsöffnung aufweist.

8. Rauchgaserzeuger nach Anspruch 7, bei dem die Sammelkammer Mittel zum Öffnen und Schließen wenigstens einer der Öffnungen aufweist.

9. Rauchgaserzeuger nach Anspruch 7 oder 8, bei dem wenigstens eine Öffnung eine Vorrichtung zum Öffnen enthält, die wiederum einen Draht enthält, dessen Gestalt temperaturabhängig ist.

10. Rauchgaserzeuger nach Ansprüche 7, 8 und 9, bei dem die Sammelkammer eine Sensoreinrichtung zum Messen der in der Sammelkammer angesammelten Rauchgasmenge hat und bei dem der Rauchgaserzeuger ferner eine Anzeigeeinrichtung hat, um in Reaktion auf ein Signal von der Sensoreinrichtung anzuzeigen, dass sich in der Sammelkammer eine vorbestimmte Rauchgasmenge befindet.

11. Rauchgaserzeuger nach einem der vorhergehenden Ansprüche, bei dem das Prüfmedium einen Festkörper oder ein gelartiges Material enthält.

12. Rauchgaserzeuger nach einem der vorhergehenden Ansprüche, bei dem das Rauchgas Rauch ist.

13. Rauchgaserzeuger nach einem der vorhergehenden Ansprüche, bei dem die Gebläseeinrichtung die Aufgabe hat, die Luft pulsierend zu beschleunigen.

14. Rauchgaserzeuger nach einem der vorhergehenden Ansprüche, bei dem die Gebläseeinrichtung ein Ventilator ist.

15. Rauchgaserzeuger nach einem der vorhergehenden Ansprüche, ferner umfassend eine Batterie oder eine wiederaufladbare Batterie zum Versorgen des Generators mit Energie.

16. Verfahren zum Erzeugen eines Rauchgases in einem mobilen Rauchgaserzeuger nach einem der Ansprüche 1-15, wobei das Verfahren Folgendes umfasst:
Zuführen eines Prüfmediums zu einer Heizeinrichtung,
Erhitzen des Prüfmediums, wodurch ein Rauchgas erzeugt wird,
Aktivieren eines Gebläses zum Umwälzen von Luft im Rauchgaserzeuger,
Ausstoßen des Rauchgases aus dem Rauchgaserzeuger.

17. Verfahren nach Anspruch 16, bei dem das erzeugte Rauchgas in einer Sammelkammer gesammelt wird, bevor es aus dem Rauchgaserzeuger ausgestoßen wird.

18. Verfahren nach Anspruch 16, bei dem das Rauchgas aus dem Rauchgaserzeuger ausgestoßen wird, nachdem sich eine vorbestimmte Rauchgasmenge in der Sammelkammer angesammelt hat.

19. Verfahren nach einem der Ansprüche 16 bis 18, ferner umfassend das Bringen des mobilen Rauchgaserzeugers in enge Nähe zu einem Rauchgasmelder.

## Claims

1. A mobile flue gas generator comprising:
a housing (38) including an electrical heating means (9,44), a supply means (39), and blowing means (23,32,41), the supply means arranged to supply a test medium (50) to the heating means, wherein the heating means is arranged to heat the test medium in order to produce a flue gas (25,33), and the blowing means is adapted to provide a circulation of air in the generator and release the generated flue gas from the generator through an opening (24,59,68).

2. The generator of claim 1 further comprising a heat chamber (42) to house the heating means and the blowing means, the heat chamber comprising an opening (59).

3. The generator of claim 2 wherein the generated flue gas is at least temporarily accumulated in the heat chamber.

4. The generator of claim 3 wherein the heat chamber comprises means for opening and closing the opening.

5. The generator of claim 1, further comprising an accumulation chamber for at least temporarily accumulating the generated flue gas.

6. The generator of claim 2 further comprising an accumulation chamber for at least temporarily accumulating the generated flue gas, wherein the accumulation chamber is in communication with the opening of the heat chamber.

7. The generator of claim 5 or 6 wherein the accumulation chamber has at least one inlet opening and/or at least one exit opening.

8. The generator of claim 7, wherein the accumulation chamber comprises means for opening and closing at least one of the openings.

9. The generator of claim 7 or 8 wherein at least one opening contains an opening device which in turn contains a wire, the form of which is temperature dependent.

10. The generator of claims 7, 8 and 9 wherein the accumulation chamber comprises a sensing means for sensing the amount of flue gas accumulated in the accumulation chamber, and wherein the generator further comprises an indication means for indicating that a predetermined amount of flue gas is present in the accumulation chamber in response to a signal from the sensing means.

11. The generator of any of the preceding claims wherein the test medium contains a solid or gel like material.

12. The generator of any of the preceding claims wherein the flue gas is smoke.

13. The generator of any of the preceding claims wherein the blowing means is adapted to accelerate the air in a pulse-like manner.

14. The generator of any of the preceding claims wherein the blowing means is a fan.

15. The generator of any of the preceding claims further comprising a battery or rechargeable battery to provide power to the generator.

16. A method for generating a flue gas in a mobile flue gas generator according to any of claims 1 to 15, the method comprising:
supplying a test medium to a heating means;
heating the test medium thereby producing a flue gas;
activating a blower to circulate air in the generator;
expelling the flue gas from the generator.

17. The method of claim 16, wherein the generated flue gas is accumulated in an accumulation chamber before it is expelled from the generator.

18. The method of claim 16, wherein the flue gas is expelled from the generator after a predetermined amount of flue gas has accumulated in the accumulation chamber.

19. The method of any one of claims 16 to 18 further comprising bringing the mobile flue gas generator into close proximity with a flue gas detector.

## Revendications

1. Générateur de gaz de carneau mobile comprenant :
un logement (38) comprenant un moyen de chauffage électrique (9, 44), un moyen d'alimentation (39) et des moyens de soufflage (23, 32, 41), le moyen d'alimentation étant agencé pour fournir un milieu d'essai (50) au moyen de chauffage, dans lequel le moyen de chauffage est agencé pour chauffer le milieu d'essai afin de produire un gaz de carneau (25, 33) et le moyen de soufflage est adapté pour fournir une circulation d'air dans le générateur et libérer le gaz de carneau généré du générateur à travers une ouverture (24, 59, 68).

2. Générateur selon la revendication 1, comprenant en outre une chambre thermique (42) pour loger le moyen de chauffage et les moyens de soufflage, la chambre thermique comprenant une ouverture (59).

3. Générateur selon la revendication 2, dans lequel le gaz de carneau généré est au moins temporairement accumulé dans la chambre thermique.

4. Générateur selon la revendication 3, dans lequel la chambre thermique comprend des moyens pour l'ouverture et la fermeture de l'ouverture.

5. Générateur selon la revendication 1, comprenant en outre une chambre d'accumulation pour accumuler au moins temporairement le gaz de carneau généré.

6. Générateur selon la revendication 2, comprenant en outre une chambre d'accumulation pour accumuler au moins temporairement le gaz de carneau généré, dans lequel la chambre d'accumulation est en communication avec l'ouverture de la chambre thermique.

7. Générateur selon la revendication 5 ou 6, dans lequel la chambre d'accumulation possède au moins une ouverture d'entrée et/ou au moins une ouverture de sortie.

8. Générateur selon la revendication 7, dans lequel la chambre d'accumulation comprend des moyens pour l'ouverture et la fermeture d'au moins l'une des ouvertures.

9. Générateur selon la revendication 7 ou 8, dans lequel au moins une ouverture contient un dispositif d'ouverture qui contient à son tour un fil métallique dont la forme dépend de la température.

10. Générateur selon les revendications 7, 8 et 9, dans lequel la chambre d'accumulation comprend un moyen de détection pour la détection de la quantité de gaz de carneau accumulée dans la chambre d'accumulation, et dans lequel le générateur comprend en outre un moyen d'indication pour l'indication qu'une quantité prédéterminée de gaz de carneau est présente dans la chambre d'accumulation en réponse à un signal provenant du moyen de détection.

11. Générateur selon l'une quelconque des revendications précédentes, dans lequel le milieu d'essai contient un matériau de type solide ou gel.

12. Générateur selon l'une quelconque des revendications précédentes, dans lequel le gaz de carneau est de la fumée.

13. Générateur selon l'une quelconque des revendications précédentes, dans lequel le moyen de soufflage est adapté pour accélérer l'air d'une manière semblable à une impulsion.

14. Générateur selon l'une quelconque des revendications précédentes, dans lequel le moyen de soufflage est un ventilateur.

15. Générateur selon l'une quelconque des revendications précédentes, comprenant en outre une pile ou une pile rechargeable pour fournir de la puissance au générateur.

16. Procédé de génération d'un gaz de carneau dans un générateur de gaz de carneau mobile selon l'une quelconque des revendications 1 à 15, le procédé comprenant :
la fourniture d'un milieu d'essai à un moyen de chauffage ; le chauffage du milieu d'essai, produisant de ce fait un gaz de carneau ;
l'activation d'une soufflante pour faire circuler de l'air dans le générateur ;
l'évacuation du gaz de carneau du générateur.

17. Procédé selon la revendication 16, dans lequel le gaz de carneau généré est accumulé dans une chambre d'accumulation avant d'être évacué du générateur.

18. Procédé selon la revendication 16, dans lequel le gaz de carneau est évacué du générateur après qu'une quantité prédéterminée de gaz de carneau s'est accumulée dans la chambre d'accumulation.

19. Procédé selon l'une quelconque des revendications 16 à 18, comprenant en outre l'amenée du générateur de gaz de carneau mobile à proximité immédiate d'un détecteur de gaz de carneau.
